# EUROPEAN PATENT APPLICATION

(11) **EP 4 706 703 A1**
(43) Date of publication of application: **11.03.2026**
(21) Application number: 25200614.3
(22) Date of filing: 05.09.2025
(51) Int. Cl.: A61M 1/16

(54) **OXYGEN RESERVE FOR ECMO THERAPY**

(30) Priority: 06.09.2024 US 202463691381 P
(71) Applicant: Breethe, Inc., Halethorpe, MD 21227 (US)
(72) Inventor: COURNANE, Stephen, Halethorpe, US-21227 (US); ZHANG, Jiafeng, Halethorpe, US-21227 (US)
(74) Representative: ABG Intellectual Property Law, S.L.

(57) **Abstract**

An extracorporeal membrane oxygenation (ECMO) system for infusing blood with oxygen and for removing carbon dioxide from blood includes a mobile oxygen storage device to provide oxygen to an oxygenator while the patient is either stationary or in ambulation. While the patient is stationary, the oxygen storage device supplies oxygen to the oxygenator for a wall oxygen supply, and excess oxygen from the wall supply additionally refills a reserve oxygen tank. While the patient is ambulating, the oxygen storage device supplies oxygen to the oxygenator for an oxygen concentrator that is supplemented from the oxygen tank, and excess oxygen from the concentrator additionally refills the oxygen tank.

## Description

### CROSS-REFERENCE TO RELATED APPLICATIONS

This application claims the benefit of the filing date of United States Provisional Patent Application No. 63/691,381 filed September 6, 2024, the disclosure of which is hereby incorporated herein by reference.

### BACKGROUND

The present disclosure relates to the recovery of a patient undergoing extracorporeal membrane oxygenation (ECMO), sometimes also referred to as extracorporeal life support (ECLS), and more particularly to a system for sharing oxygen from extracorporeal oxygenation for other use by a recovering patient.

Patients awaiting or recovering from a heart or lung transplant and patients having conditions putting them at risk for heart or lung failure may be candidates for ECMO therapy. ECMO therapy is designed to address an excess of carbon dioxide and/or lack of oxygen in the blood where the lungs are not healthy and/or the heart cannot pump enough blood around the body. ECMO therapy is thus an extracorporeal technique which replaces these respiratory and cardiac functions in various situations ranging from support needed while separately treating the underlying causes of cardiac arrest to late-stage treatment for heart or lung failure. It may be a therapeutic treatment to provide temporary help when needed.

ECMO therapy operates by drawing deoxygenated blood from the body of a patient in a controlled manner to permit oxygenation of the blood and to remove carbon dioxide from the blood. The oxygenated blood is then cycled back into the patient. Generally, ECMO systems include a pump, an oxygenator, an oxygen source and a control center to monitor and control the process. It may also include a blood warmer to bring the blood back up to the temperature of the body as it is circulated back into the body. Deoxygenated blood drawn from the body via a cannula is pumped through an oxygenator where oxygen is infused in the blood and carbon dioxide is removed as a result of such infusion. The oxygenated blood is pumped from the oxygenator and back into the body, with blood warming in most instances.

### BRIEF SUMMARY

In accordance with a first aspect of the present disclosure, a blood oxygenation system includes an oxygenator configured to oxygenate blood of a patient, a pump for pumping oxygen into the oxygenator, and an oxygen storage device for supplying oxygen to the oxygenator. The oxygen storage device comprises a first oxygen supply and a second oxygen supply each configured to selectively supply oxygen to the oxygenator.

In another aspect of the present disclosure, the first storage device may be an oxygen concentrator. The oxygen concentrator may be configured to store oxygen received from atmospheric air. The oxygen concentrator may be configured to remove nitrogen from the atmospheric air. The oxygen concentrator may supply oxygen to the oxygenator at a rate of 3 liters per minute.

In yet another aspect of the present disclosure, the second storage device may be an oxygen tank. The oxygen tank may have a volume of 25 cubic inches. The oxygen tank may supplement the supply of oxygen from first oxygen supply to the oxygenator.

In yet another aspect of the present disclosure, the blood oxygenation system may include a blood pump for pumping deoxygenated blood into the oxygenator.

In yet another aspect of the present disclosure, a method of administering oxygen to blood of a patient comprises the steps of directing the blood from a patient to an oxygenator, supplying oxygen to the oxygenator from an oxygen storage device to infuse the blood with oxygen, and controlling the oxygen supply to the oxygenator between the patient being in a first environment and the patient being in a second environment different from the first environment.

In yet another aspect of the present disclosure, controlling the oxygen supply to the oxygenator in the first environment occurs while the oxygen storage device is stationary. Controlling the oxygen supply to the oxygenator in the first environment may occur while the patient is stationary. Controlling the oxygen supply to the oxygenator in the first environment may be supplied from a wall oxygen supply.

In yet another aspect of the present disclosure, the method may further comprise the step of refilling an oxygen tank with excess oxygen supplied from the wall oxygen supply in the first environment. Controlling the oxygen supply to the oxygenator in the second environment may occur when the oxygen storage device is mobile. Controlling the oxygen supply to the oxygenator in the second environment may occur while the patient is ambulating. Supplying oxygen to the oxygenator in the second environment may be supplied from an oxygen concentrator and an oxygen tank. Supplying oxygen to the oxygenator in the second environment may be received primarily from the oxygen concentrator and may be supplemented as needed by the oxygen tank. The method may further comprise the step of refilling the oxygen tank with excess oxygen supplied from the oxygen concentrator in the second environment.

### BRIEF DESCRIPTION OF THE DRAWINGS

The drawings described herein are for illustrative purposes only of the selected embodiments and are not all possible implementations and thus are not intended to limit the scope of the present disclosure.
FIG. 1 is a schematic representation of an ECMO system in accordance with an embodiment of the present disclosure;
FIG. 2 is a schematic representation of an oxygen source for the EMCO system of FIG. 1; and
FIG. 3 is a schematic representation of an oxygen tank of the oxygen source of FIG. 2.

### DETAILED DESCRIPTION

Patients awaiting or recovering from a heart or lung transplant and patients having conditions putting them at risk for heart or lung failure may be candidates for ECMO therapy. ECMO therapy is designed to address an excess of carbon dioxide and/or lack of oxygen in the blood where the lungs are not healthy and/or the heart cannot pump enough blood around the body. ECMO therapy is thus an extracorporeal technique which replaces these respiratory and cardiac functions in various situations ranging from support needed while treating the underlying causes of cardiac arrest to late-stage treatment for heart or lung failure. It is a therapeutic treatment to provide temporary help when needed. It does not address the underlying condition.

ECMO systems generally receive oxygen from a hospital wall supply or another stationary oxygen source. However, these oxygen wall supplies are only used when the patient is stationary, such as in a hospital bed. Due to the importance of ECMO therapy, there are situations when a patient's ambulation is necessary and highly desirable, and mobile oxygen sources are needed for these ECMO systems.

During ECMO therapy, a patient's ambulation is often cumbersome due in part to the requirement for a mobile oxygen source. As a result, the patient is saddled with considerable equipment as the patient moves about. This includes the oxygen concentrator, oxygen tank, additional mobile oxygen source components, and other systems critical to the care of the patient. As ambulation is itself critical to recovery and mental well-being, it is desirable to unburden the patient from medical baggage to enable the patient to move about more freely and comfortably.

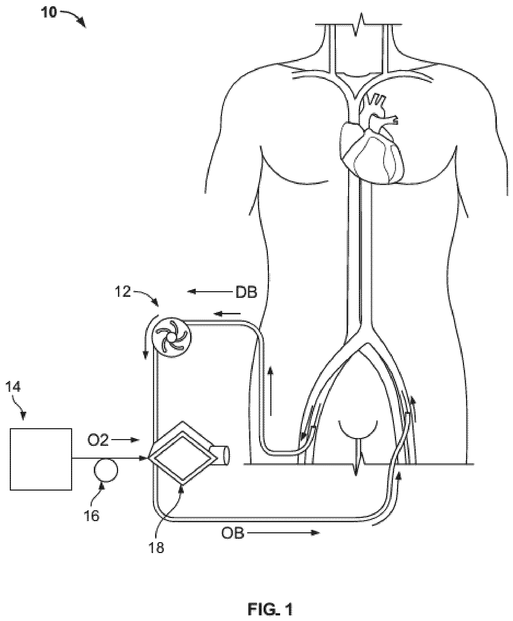

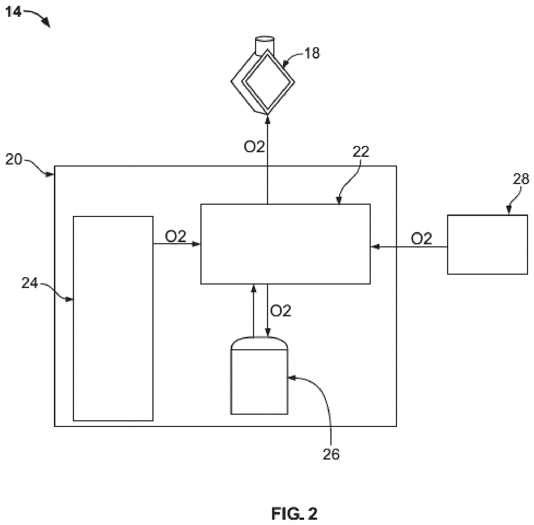

Referring to FIG. 1, an ECMO system 10 is shown which includes a blood pump 12, an oxygen source 14, an oxygen pump 16 and an oxygenator 18. Shown more specifically in FIG. 2, the oxygen source 14 includes a housing 20, a gas management system 22, an oxygen concentrator 24 and an oxygen tank 26. The oxygen concentrator 24 extracts and filters oxygen from ambient air, while the oxygen tank 26 supplies stored oxygen. Additionally, as discussed further herein, oxygen source 14 may be connected to a wall oxygen supply 28. The present disclosure contemplates any suitable arrangement of an oxygen source. By way of example, any suitable gas management system, oxygen concentrator and/or oxygen tank may be used within oxygen source or connected external to oxygen source.

ECMO system 10 allows deoxygenated blood DB to be pumped into a blood inlet of the oxygenator 18, while pressurized oxygen O2 is pumped into an oxygen inlet of the oxygenator 18. As shown in FIG. 2, this oxygen O2 flows from the oxygen source 14 into oxygenator 18. Specifically, gas management system 22 controls the flow of oxygen O2 from the concentrator 24, tank 26 and wall supply 28 to the oxygenator 18. Concentrator 24 may supply oxygen O2 to oxygenator 18 at a rate of 3 liters per minute, while tank 26 may have a volume of 25 cubic inches and supply oxygen O2 to the oxygenator 18 at a similar rate as concentrator 24. After sufficient oxygen O2 has been received by oxygenator 18, deoxygenated blood DB is infused with oxygen O2 such that oxygenated blood OB exits the oxygenator 18 at a blood outlet and is circulated back into the patient. As will be appreciated, concentrator 24 may supply oxygen O2 to the oxygenator 18 at any suitable rate. As will also be appreciated, tank 26 may have any suitable volume and may supply oxygen O2 to the oxygenator 18 at any suitable rate.

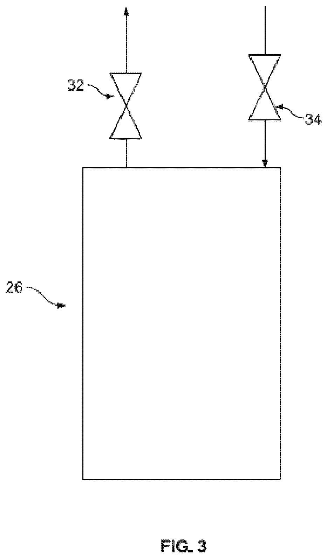

In use, oxygen source 14 provides a hybrid approach for supplying oxygen O2 to oxygenator 18 of the ECMO system 10. When oxygen source 14 is connected to wall supply 28, gas management system 22 controls the flow of oxygen O2 such that wall supply 28 supplies oxygen O2 directly to oxygenator 18. Simultaneously, excess oxygen O2 from wall supply 28 not needed or necessary to be received by oxygenator 18 will be redirected to refill or recharge tank 26. In other words, when oxygen source 14 is connected to wall supply 28, oxygen O2 will not flow from concentrator 24 or tank 26 to oxygenator 18 while oxygen O2 from wall supply 28 will both supply oxygenator 18 and, if necessary, refill tank 26. For example, as shown in FIG. 3, the connection between gas management system 22 and tank 26 and includes an outlet valve 32 and a fill valve 34. When oxygen source 14 is connected to wall supply 28, gas management system 22 causes fill valve 32 to open and outlet valve 34 to close, which allows excess external oxygen to flow from wall supply 28 through the opened fill valve 34 to tank 26. Additionally, as the outlet valve 34 remains closed when oxygen source 14 is connected to wall supply 28, oxygen will be prevented from flowing from oxygen tank 26 to oxygenator 18, and all oxygen supplied to oxygenator 18 will flow directly from wall supply 28.

In another use, oxygen source 14 is not connected to wall supply 28 and provides a mobile supply of oxygen O2 to oxygenator 18. Specifically, gas management system 22 controls the flow of oxygen O2 such that concentrator 24 primarily supplies oxygen O2 to oxygenator 18. If there is excess oxygen O2 from concentrator 24 not needed or necessary to be received by oxygenator 18, excess oxygen O2 will be redirected to refill or recharge tank 26. However, if gas management system 22 determines the flow of oxygen O2 solely from concentrator 24 to oxygenator 18 is insufficient, supplemental oxygen O2 is supplied from tank 26. For example, as shown in FIG. 3, when oxygen source 14 is disconnected from wall supply 28, gas management system 22 will detect that there is no external oxygen flow and cause both outlet valve 32 and fill valve 34 to close. Additionally, with no external oxygen flow detected, gas management system 22 will activate concentrator 24 to directly supply oxygenator 18. With both outlet valve 32 and fill valve 34 closed, oxygen will be prevented from flowing from concentrator 24 to tank 26 and any stored oxygen in tank 26 will be prevented from flowing to oxygenator 18. Gas management system 22 will continue to maintain outlet valve 32 in a closed position as oxygen is supplied from concentrator 24 to oxygenator 18 at a predetermined rate, such as 3 liters per minute. However, if gas management system 22 determines an increase in the oxygen supply to oxygenator 18 is necessary, gas management system 22 will variably open outlet valve 32 to allow oxygen stored in tank 26 to flow to oxygenator 18. For example, if gas management system 22 determines oxygenator 18 requires a flow rate greater than the predetermined rate, valve 32 is variably opened to allow tank 26 to supplement or increase the oxygen flow rate to oxygenator 18 at a variable rate, such as between 0 to 3 liters per minute.

Mobile oxygen sources for ECMO systems are desirable to ambulate patients when the ECMO system is not connected to a wall oxygen supply. Current mobile oxygen sources typically use oxygen concentrators and oxygen tanks, but these oxygen concentrators are too small and have limited flow rates insufficient for patient ambulation. Alternatively, larger oxygen concentrators require larger sieves, pumps and additional power, making larger concentrators difficult to transport and maintain within a mobile housing. Further, the tanks used with current mobile oxygen sources are too large and cumbersome to be used and transported efficiently, and these tanks require constant replacement once all available oxygen has been supplied to the oxygenator.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A blood oxygenation system, comprising:
an oxygenator configured to oxygenate blood of a patient;
a pump for pumping oxygen into the oxygenator;
an oxygen storage device for supplying oxygen to the oxygenator, wherein the oxygen storage device comprises a first oxygen supply and a second oxygen supply each configured to selectively supply oxygen to the oxygenator.

2. The blood oxygenation system of claim 1, wherein the first oxygen supply is an oxygen concentrator, wherein the oxygen concentrator is configured to store oxygen received from atmospheric air, wherein the oxygen concentrator is configured to remove nitrogen from the atmospheric air.

3. The blood oxygenation system of any one of claims 1 or 2, wherein the oxygen concentrator supplies oxygen to the oxygenator at a rate of 3 liters per minute.

4. The blood oxygenation system of any one of the preceding claims, wherein the second oxygen supply is an oxygen tank, wherein the oxygen tank is configured to store oxygen received from a wall oxygen supply.

5. The blood oxygenation system of claim 4, wherein the oxygen tank has a volume of 25 cubic inches.

6. The blood oxygenation system of any one of claims 4 or 5, wherein the oxygen tank supplements the supply of oxygen from first oxygen supply to the oxygenator.

7. The oxygenation system of any of the preceding claims, further comprising a blood pump for pumping deoxygenated blood into the oxygenator.

8. A method of administering oxygen to blood of a patient, comprising the steps of:
directing the blood from a patient to an oxygenator;
supplying oxygen to the oxygenator from an oxygen storage device to infuse the blood with oxygen; and
controlling the oxygen supply to the oxygenator between the patient being in a first environment and the patient being in a second environment different from the first environment.

9. The method of claim 8, wherein controlling the oxygen supply to the oxygenator in the first environment occurs while the oxygen storage device is stationary.

10. The method of any one of claims 8 or 9, wherein controlling the oxygen supply to the oxygenator in the first environment occurs while the patient is stationary.

11. The method of any one of claims 8-10, wherein controlling the oxygen supply to the oxygenator in the first environment is supplied from a wall oxygen supply, further comprising the step of refilling an oxygen tank with excess oxygen supplied from the wall oxygen supply in the first environment.

12. The method of any one of claims 8-11, wherein controlling the oxygen supply to the oxygenator in the second environment occurs when the oxygen storage device is mobile.

13. The method of any one of claims 8-12, wherein controlling the oxygen supply to the oxygenator in the second environment occurs while the patient is ambulating.

14. The method of any one of claims 8-13, wherein supplying oxygen to the oxygenator in the second environment is received primarily from an oxygen concentrator and is supplemented as needed by an oxygen tank.

15. The method of claim 14, further comprising the step of refilling the oxygen tank with excess oxygen supplied from the oxygen concentrator in the second environment.
